# EUROPÄISCHE PATENTSCHRIFT

(11) **EP 2 931 343 B1**
(45) Veröffentlichungstag und Bekanntmachung des Hinweises auf die Patenterteilung: **09.09.2020**
(21) Anmeldenummer: 13805320.2
(22) Anmeldetag: 05.12.2013
(51) Int. Cl.: A61M 16/04, A61M 25/10

(54) **DRUCKAUSGLEICHSVORRICHTUNG**
PRESSURE EQUALISING DEVICE
DISPOSITIF COMPENSATEUR DE PRESSION

(30) Priorität: 11.12.2012 DE 102012112097
(43) Veröffentlichungstag der Anmeldung: 21.10.2015
(73) Patentinhaber: Tracoe Medical Gmbh, 55268 Nieder-Olm (DE)
(72) Erfinder: SCHNELL, Ralf, 63500 Seligenstadt (DE); HAHN, Andreas, 55291 Saulheim (DE)
(74) Vertreter: WSL Patentanwälte Partnerschaft mbB
(86) Internationale Anmeldenummer: PCT/EP2013/075659
(87) Internationale Veröffentlichungsnummer: WO 2014/090680

(56) Entgegenhaltungen:
- WO-A1-91/10465
- DE-A1- 3 321 155
- DE-A1- 3 433 785
- GB-A- 1 423 789
- US-A- 4 119 101

## Beschreibung

Die vorliegende Erfindung betrifft eine Druckausgleichsvorrichtung mit einem Druckausgleichsballon, wobei das Volumen des Druckausgleichsballons mit einem weiteren Volumen in Verbindung steht, dessen Druck auch bei einer aufgezwungenen Volumenänderung zumindest innerhalb eines vorgegebenen Volumenbereichs des Druckausgleichsballons einen weitgehend konstanten Wert behalten soll.

Ein solches Volumen, dessen Druck auch bei einer von außen aufgezogenen Volumenänderung zumindest innerhalb eines vorgegebenen Volumenbereichs des Druckausgleichsballons einen möglichst konstanten Wert behalten soll, ist beispielsweise das Volumen der Manschette einer Beamtungskanüle. Die Manschette, in der medizinischen Fachsprache und im Folgenden auch "Cuff" genannt, umgibt dabei eine in die Trachea (Luftröhre) eines Patienten eingeführte Kanüle und wird nach dem Einführen der Kanüle auf einen geringen Druck in der Größenordnung von 20 bis 30 mbar aufgeblasen, damit sie sich dichtend an die Wand der Trachea anlegt, die Kanüle auf diese Weise fixiert und außerdem verhindert, das Sekret, welches sich oberhalb des Cuffs in der Umgebung der Beatmungskanüle ansammelt, in die Bronchien bzw. in die Lunge des Patienten gelangt. Ansonsten könnte derartiges Sekret, in welchem sich Krankheitskeime sehr schnell vermehren und welches dann in die tieferen Atemwege oder die Lunge gelangt, zu schweren Komplikationen wie z. B. einer Lungenentzündung führen.

Eine gute Abdichtung durch einen solchen Cuff ist insbesondere bei langzeitbeatmeten Patienten von großer Bedeutung. Die Kanüle kann dabei entweder ein Endotrachealtubus sein, der durch Mund und Rachen eines Patienten eingeführt wird, kann aber ebenso gut auch eine Tracheostomiekanüle sein, die durch eine chirurgisch erzeugte Öffnung (ein Tracheostoma) im Hals eines Patienten hindurchgeführt ist und damit unter Umgehung des Mund- und Rachenraums die Trachea mit der Umgebungsluft oder einem Beatmungsgerät verbindet.

Insbesondere bei langzeitbeatmeten Patienten ist dabei auch der eingestellte Cuff-Druck von größter Bedeutung, da dieser Druck auf Dauer einen Wert von 30 mbar nicht übersteigen darf, weil der auf die sehr empfindliche Wand der Trachea wirkende Druck zu einer Behinderung der Durchblutung und im Ergebnis zu Beschädigungen bzw. Wunden bis hin zu Nekrosen führen kann.

Andererseits darf der Cuff-Druck auch nicht zu gering sein, um den Vorbeitritt des oben erwähnten Sekretes an dem Cuff vorbei zu verhindern. Außerdem könnte unkontrolliert an dem Cuff vorbeiströmende Luft auch einen Fehlalarm an einem Beatmungsgerät auslösen, da die Beatmung bei Verwendung entsprechender Kanülen grundsätzlich über das Lumen der Kanüle erfolgen soll und anhand des Luftstroms durch die Kanüle kontrolliert wird.

Um den Cuff-Druck daher auf dem im Allgemeinen als sinnvoll und optimal angesehen Druckbereich zwischen etwa 20 mbar und 30 mbar zu halten, sind bereits zahlreiche Einrichtungen verwendet und beschrieben worden, die den Druck in dem Cuff konstant halten sollen. Dabei ist zu berücksichtigen, dass z. B. durch Verschieben, Verdrehen oder Neigen des Kopfes eines Patienten auch der Tracheaquerschnitt sich ändern kann und damit in unterschiedlicher Weise auf den Cuff drückt und dessen Volumen verändert. Der Cuff muss daher über einen entsprechenden Schlauch, der im Allgemeinen an der Kanüle entlang geführt oder in die Wand der Kanüle integriert ist, mit einem entsprechenden Druckreservoir verbunden sein, welches je nach Volumenänderung des Cuffs Luft bzw. generell Füllgas wie z. B. Stickstoff, aus dem Cuff aufnehmen kann und auch wieder in den Cuff zurückführt, um den Cuff-Druck unabhängig von einem möglicherweise variierenden Cuffvolumen auf einem konstanten Wert zu halten.

Hierzu sind beispielsweise elektronische Druckregler bekannt ebenso wie auch einfache Ausgleichsvolumina, insbesondere Ballons, wobei in aller Regel der Druck eines Cuffs von medizinischem Fachpersonal in gewissen zeitlichen Abständen, oftmals jedoch nur einmal pro Schicht, kontrolliert und nachjustiert wird.

Des Weiteren ist auch bereits ein System bekannt, welches mit einem Latexballon als Ausgleichsvolumen arbeitet, wobei der Latexballon die Eigenschaft hat, zumindest innerhalb eines gewissen Volumenbereichs einen konstanten Druck aufrecht zu erhalten. Das heißt, bereits eine geringfügige Erhöhung des Drucks führt zu einer entsprechenden Volumenzunahme des Latexballons, während eine geringe Druckabnahme wieder zu einer Volumenverringerung des Latexballons führt, so dass im Ergebnis der Druck in einem Cuff, der mit einem entsprechenden Latexballon verbunden ist, auf demselben in etwa konstanten Wert bleibt. Um den sehr dünnen und empfindlichen Latexballon zu schützen, der die gewünschten Eigenschaften hat, ist er in einer äußeren Schutzhülle angeordnet, die vorzugsweise durchsichtig ist, im Wesentlichen Kugelform hat und stabil genug ist, um den in der Schutzhülle aufgenommenen Ballon gegenüber Stößen, Beschädigungen oder von außen ausgeübtem Druck zu schützen und es versteht sich, dass die Schutzhülle luftdurchlässig sein muss, um die Ausdehnung oder ein Zusammenziehen des Ausgleichsballons innerhalb der Schutzhülle nicht zu beeinflussen.

Verschiedene Vorrichtungen zur Konstanthaltung oder Überwachung des Drucks entsprechender Cuffs oder Manschetten im medizinischen Bereich sind bekannt aus den US-Patenten 3,642,005, 4,501,273, der US-Patentanmeldung 2012/0090619 A1, der EP 1 252 909 A2 und dem britischen Patent GB 1 423 789.

Elektronische Druckregelsysteme sind sehr aufwendig und teuer. Ballonsysteme mit sehr voluminösen Ausgleichsballons sind unhandlich und unterliegen der Gefahr von Beschädigungen eines solchen Ballons. Das bekannte System mit einem Ausgleichsballon aus Latex, der sich innerhalb einer stabilen, durchsichtigen Schutzhülle befindet, funktioniert im Prinzip relativ gut, hat aber den Nachteil, dass das Latex u. a. aufgrund seines allergenen Potentials in vielen Bereichen der medizinischen Behandlung unerwünscht oder gar unzulässig ist.

Die EP 1 252 909 A2 beschreibt bereits ein System aus einem Hauptballon (entsprechend dem Cuff) und einem Pilotballon, wobei Hauptballon und Pilotballon dieselbe Elastizität aufweisen sollen, so dass der Zustand oder auch insbesondere der Druck des Hauptballons (Cuff) anhand des Zustandes des sichtbaren Pilotballons ablesbar ist. Die EP 1 252 909 beschreibt dabei sowohl für den Hauptballon als auch für den Pilotballon ein Material aus thermoplastischen Elastomeren auf Styrolbasis.

Auch wenn der Druck in dem Cuff einer Beatmungskanüle in der Regel konstant gehalten werden soll, gibt es Situationen im medizinischen Alltag, in welchen bewusst und im Allgemeinen nur für kurze Zeit ein solcher Cuff mit einem höheren Druck beaufschlagt werden soll, der beispielsweise zwischen 30 und 50 mbar liegt. Dies ist mit den vorgenannten Druckausgleichsballons, die sich bei nur geringfügig zunehmendem Druck immer weiter ausdehnen, praktisch kaum zu realisieren. Zwar ist es bekannt, einen solchen Ballon mit einer festen Schutzhülle zu versehen, die ein weiteres Dehnen übder das Volumen der Schutzhülle hinaus verhindert. Jedoch bedeutet dies, dass der Druck plötzlich sehr stark ansteigt, sobald sich der Ausgleichsballon an die Innenwand der Schutzhülle angelegt hat. Eine gut kontrollierte Erhöhung auf einen nur leicht erhöhten Druck von z. B. etwa 30 bis 50 mbar ist dabei kaum möglich.

WO 91/10465 A1 beschreibt eine Druckausgleichsvorrichtung mit einer scheibenförmigen Schutzhülle und mit einem im Inneren der Schutzhülle angeordneten Ausgleichballon.

US 4,119,101 A beschreibt eine Druckausgleichsvorrichtung mit einer quaderförmigen Schutzhülle und mit einem im Inneren der Schutzhülle angeordneten Ausgleichballon. Das Innere der Schutzhülle wird über ein Atemgerät entsprechend dem Atemzyklus mit einem wechselnden Druck beaufschlagt.

Gegenüber diesem Stand der Technik liegt der vorliegenden Erfindung die Aufgabe zugrunde, eine Druckausgleichsvorrichtung bereitzustellen, welche einerseits einen vorgegebenen Druck in einem anderen, an den Ausgleichsballon angeschlossenen Volumen, wie zum Beispiel einem Cuff bzw. einer Manschette, im Wesentlichen konstant hält, es jedoch auch ermöglicht, den Druck bei Bedarf kontrolliert zu erhöhen. Diese Aufgabe ist beim Gegenstand des Anspruchs 1 gelöst.

Diese Aufgabe wird dadurch gelöst, dass der den Druck im Allgemeinen konstant haltende Ausgleichsballon in einer Schutzhülle aufgenommen ist, die abweichend von einer gleichmäßig konkaven, der äußeren Ballonkontur angepassten Form radiale Ausbuchtungen aufweist, die so gestaltet sind, dass der Ausgleichsballon sich zunächst an die der Kontur des Ballons angepassten Wandbereiche der Schutzhülle anlegt und anschließend bei Druckerhöhung nur in die Ausbuchtungen hinein ausdehnt, wodurch der Druck in dem Ausgleichsballon mit der Volumenzunahme in die Ausbuchtungen hinein allmählich ansteigt, wobei die Schutzhülle eine kugelförmige Grundform mit streifenförmigen Ausbuchtungen.

Zweckmäßig ist es dabei, wenn in dem Volumenbereich des Ausgleichsballons zwischen dem Volumen bei Anlage an den der Kontur des freien Ballons angepassten Bereichen und dem Volumen bei vollständigem Ausfüllen aller Ausbuchtungen allmählich ansteigt und zwar vorzugsweise in etwa um den Faktor zwei.

Die Volumendifferenz zwischen dem Volumen bei Anlage an den der Kontur des freien Ballons angepassten Bereichen und dem Volumen bei vollständigem Ausfüllen aller Ausbuchtungen sollte etwa bei mindestens 5 % des erstgenannten Volumens liegen. Der relative Flächenanteil der konkaven Ausbuchtungen 6 zu den konvexen Abschnitten 5a kann zum Beispiel zwischen 20 % und 70 % liegen.

In einer bevorzugte Ausführungsform ist der Ausgleichsballon aus einem thermoplastischen Polymer hergestellt und wurde vor dem ersten Gebrauch über das maximal vorgesehene Regelvolumen hinaus überdehnt und anschließend bis auf den Bereich des Regelvolumens relaxiert, wie dies in der gleichzeitig eingereichten Patentanmeldung desselben Anmelders mit dem Titel "Druckausgleichsballon und Verfahren zu dessen Herstellung" beschrieben wird.

Zweckmäßigerweise wird ein entsprechender Ausgleichsballon für die Verwendung an Beatmungskanülen mit einem Anfangsvolumen (vor dem Überdehnen) von etwa 3 bis 8 cm³ herge stellt, welches sich nach dem erstmaligen Überdehnen, z. B. auf ein Volumen von etwa 200 cm³ (oder auch mehr), um etwa 20 bis 50% vergrößert hat. Das Regelvolumen könnte dann im Bereich von z. B. 10 oder 20 bis 100 cm³ liegen, vorzugsweise im Bereich von 30 bis 70 cm³.

Die vorliegende Erfindung ist aber nicht auf einen solchen Ballon aus thermoplastischen Polymeren beschränkt sondern auf alle Arten von Ballonen anwendbar, die eine entsprechende Druckcharakteristik haben, die zunächst keinen oder fast keinen Druckanstieg mit zunehmendem Volumen zeigt, deren Druck aber ab einem bestimmten Volumen deutlicher zunehmen soll.

Der Ausgleichsballon ist von einer Schutzhülle umgeben, die den relativ empfindlichen Ausgleichsballon vor äußeren mechanischen Einwirkungen schützt. Eine solche Schutzhülle, z. B. aus stabilem, durchsichtigem Kunststoffmaterial hat eine kugelförmige Grundform mit Ausbuchtungen bzw. Ausstülpungen nach außen. Die Schutzhülle kann zum Beispiel einen Durchmesser in der Größenordnung von 50 bis 60 mm haben.

Wie bereits erwähnt, könnte eine entsprechende kugelförmige Schutzhülle aus Plexiglas oder irgendeinem anderen geeigneten, stabilen Kunststoffmaterial einen Durchmesser in der Größenordnung von 50 bis 60 mm und damit ein Volumen in der Größenordnung von 70 bis 100 cm³ haben. Die Schutzhülle kann eventuell auch kleiner gehalten werden und einen Durchmesser von 40 mm und bis herab zu 30 mm oder weniger aufweisen. Es versteht sich, dass die entsprechende Schutzhülle mindestens eine Öffnung aufweist, die das Innere der Schutzhülle mit der Umgebung verbindet, um außerhalb des Ausgleichsballons immer den konstanten Umgebungsdruck aufrecht zu erhalten, so dass der Ausgleichsballon sich innerhalb des Volumens der Schutzhülle ungehindert ausdehnen und zusammenziehen kann.

Dabei ist jedoch vorgesehen, dass die Schutzhülle, ausgehend von Ihrer kugelförmigen Grundform, einige, streifenartig angeordnete, Ausbuchtungen aufweist. Dies führt beispielsweise dazu, dass der Ausgleichsballon sich bei einem geringfügigen Druckanstieg zunächst ausdehnt, bis er an Teilen der Innenwand der Schutzhülle anliegt, während danach eine weitere Dehnung des Ausgleichsballons nur noch im Bereich der Ausbuchtungen möglich ist. Dies bewirkt einen geringen aber deutlich spürbaren und sichtbaren Druckanstieg, bis auch die Ausbuchtungen mit dem Ballon ausgefüllt sind, woraufhin der Druck bei weiterer Gaszufuhr steil ansteigt. In dem Übergangsbereich, in welchem der Ballon sich in die Ausbuchtungen hinein ausdehnt, ist jedoch ein verhältnismäßig leichter, kontinuierlicher Druckanstieg festzustellen, der es ermöglicht, gezielt und kontrolliert einen zeitweise gewünschten, höheren Cuff-Druck einzustellen. Dies ist beispielsweise in manchen Notsituationen erforderlich, wenn z. B. Blutungen in der Trachea bzw. im Hals- und Rachenraum auftreten und gestillt werden müssen, ohne dass die dichte Anlage des Cuffs an der Tracheawand aufgegeben wird, sondern ggf. sogar verstärkt werden muss

Ein besonderer Vorteil der Verwendung von thermoplastischen Polymeren liegt darin, dass ein entsprechender Ballon im Spritzgussverfahren hergestellt werden kann, was eine sehr gut kontrollierbare, gleichmäßige Wandstärke ermöglicht, wobei die Wandstärke wiederum einer der Parameter ist, durch welche der konkrete Regeldruck eingestellt werden kann. Ein zweiter Parameter für die Einstellung eines Regeldrucks liegt in dem Ausmaß der Überdehnung.

Als besonders geeignet haben sich styrolbasierte thermoplastische Polymere erwiesen, wie z. B. SBS (Styrol-Butadien-Styrol), SIS (Styrol-Isopren-Styrol) und SEPS (Styrol-Ethylen-Propylen-Styrol). Besonders bevorzugt ist für die vorliegende Erfindung das Material SEBS (Styrol-Ethylen-Butylen-Styrol).

Das thermoplastische Material kann auch Füllstoffe, wie zum Beispiel Mineralöle und insbesondere medizinische Weißöle aufweisen.

Zweckmäßigerweise wird das thermoplastische Material so ausgewählt bzw. eingestellt, dass es eine Shore 00 Härte (nach ISO 868) von 30 - 70, vorzugsweise 50 Shore 00 hat. Der Zugmodul bei 100 % Dehnung liegt typischerweise im Bereich zwischen 40-150 kPa (nach ISO 37).

Die Wandstärke des drucklosen Ballons vor dem erstmaligen Überdehnen, d. h. nach dem Spritzgießen, kann z. B. im Bereich zwischen 0,4 und 1,5 mm liegen. Beim Dehnen eines solchen Ballons und insbesondere zum Überdehnen wird typischerweise ein Druck in der Größenordnung von 40 bis 60 mbar benötigt. Lässt man jedoch den Ballon nach einem hinreichenden Überdehnen wieder relaxieren und zurück in den drucklosen Zustand gelangen, so hat er in diesem Zustand zum einen ein im Allgemeinen etwas größeres Volumen als nach dem Spritzgießen, vor allem aber ein anderes Dehnungsverhalten, d. h. der Druck, der zum Aufblähen des Ballons innerhalb des Regelvolumenbereichs (typischerweise weniger als ein Viertel des Überdehnungsvolumens) benötigt wird, liegt jedoch nur noch bei etwa der Hälfte des Druckes, der für das erstmalige Dehnen und Überdehnen des Ballons erforderlich ist und er bleibt über einen größeren Volumenbereich des Ballons hinweg weitgehend konstant. Wie gesagt kann der genaue Wert durch gezielte Wahl der Wandstärke und durch das kontrollierte Ausmaß der Überdehnung relativ genau eingestellt werden.

Zwar zeigt das Material gelegentlich einen gewissen "Erholungseffekt", wenn es längere Zeit im drucklosen Zustand gehalten wurde, was dazu führt, dass beim erstmaligen Wiederaufblähen der Regeldruckwert etwas überschritten werden muss; wird jedoch das zur Verfügung stehende Regelvolumen (welches beispielsweise durch die Schutzhülle begrenzt wird) vor der in Betriebnahme noch einmal vollständig ausgenutzt, so stellt sich das gewünschte Dehnungsverhalten bei dem Regeldruck sehr schnell wieder ein.

Ein weiterer Vorteil der Herstellung mittels Spritzgussverfahren ist es, dass der Ausgleichsballon an verschiedenen Stellen wahlweise mit unterschiedlichen Wandstärken hergestellt werden kann. Insbesondere ist in einer bevorzugten Ausführungsform der Erfindung vorgesehen, dass der Ausgleichsballon eine Ballonöffnung umgebenden verstärkten Öffnungsring aufweist, dessen Wandstärke mindestens 50% größer ist als die Wandstärke des Ballons im übrigen und der dementsprechend an der Dehnung beim Druckausgleich nicht teilnimmt. Ein solcher Öffnungsring kann mit einer genau definierten Geometrie und Wandstärke hergestellt werden, so dass er beispielsweise unmittelbar auf einen entsprechenden Standardanschluss (z. B. Luer-Connector) in dichtem Eingriff aufgesetzt werden kann.

Der Öffnungsring kann insbesondere auch einen umlaufenden Dichtungsflansch aufweisen, der an einem entsprechenden Anschlussstutzen der Verbindungsleitung zu einem Cuff leicht fixiert werden kann.

Entsprechende Standardanschlüsse haben typischerweise einen Durchmesser im Bereich von 12 bis 25 mm.

Der Ballon sollte zweckmäßigerweise so hergestellt werden, dass das nutzbare Regelvolumen den Bereich von 10 bis 100 cm³, zweckmäßigerweise den Bereich von 20 bis 80 cm³ umfasst. Das maximale Regelvolumen wird zweckmäßigerweise begrenzt durch eine stabile, durchsichtige äußere Schutzhülle. Dadurch ist der Ballon im Inneren der durchsichtigen Hülle sichtbar und das Fachpersonal, welches die entsprechenden Beatmungsvorrichtungen bedient, kann anhand des Zustandes des Ballons unmittelbar erkennen, ob der Druck der Manschette bzw. des Cuffs im gewünschten Bereich liegt.

Weitere Vorteile, Merkmale und Anwendungsmöglichkeiten der vorliegenden Erfindung ergeben sich aus der folgenden Beschreibung einer bevorzugten Ausführungsform und der dazugehörigen Figuren.

Es zeigen:
- Figur 1: im linken Teilbild eine perspektivische Ansicht und im rechten Teilbild eine vertikale Schnittansicht durch einen erfindungsgemäßen Ausgleichsballon im drucklosen Zustand, wie er zum Beispiel unmittelbar nach der Herstellung im Spritzgussverfahren vorliegt,
- Figur 2: einen Druckausgleichsballon im aufgeblähten Zustand wie er während der Druckregelung vorliegt, wobei die Kontur des drucklosen Ballons ebenfalls wiedergegebene ist, und
- Figur 3: einen Querschnitt durch den Ballon gemäß Figur 2 entsprechend der Linie III-III mit einer zusätzlich um den Ballon angeordneten Schutzhülle.
- Figur 4: eine schematische Ansicht auf eine weitere Ausführungsform der vorliegenden Erfindung,
- Figur 5:: eine Schnittansicht der Ausführungsform nach Figur 4 entsprechend der Schnittlinie V-V in Figur 4 und
- Figur 6:: eine Schnittansicht ähnlich der Figur 5 welche nicht Teil der Erfindung ist.

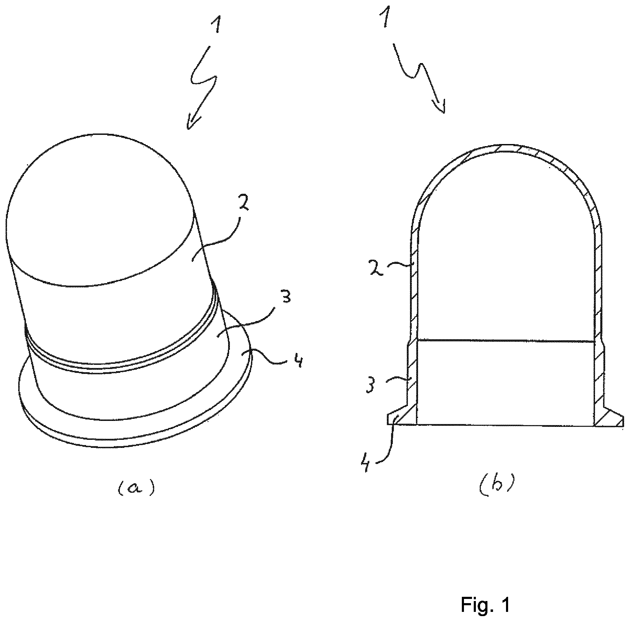

In Figur 1 erkennt man einen Druckausgleichsballon 1, der aus einem kuppelförmigen Dehnungsabschnitt 2, einem zylindrischen Abschnitt 3 mit etwas größerer Wandstärke und schließlich einem Flanschabschnitt 4 besteht, wobei die Abschnitte 3 und 4 einen Öffnungs- und Anschluss-Abschnitt des Druckausgleichsballons 1 bilden. Beispielsweise können die Abschnitte 3 und 4 auf einen im medizinischen Bereich typischen Luuer-Konnektor, d.h. einen Rohrstutzen mit dem passenden Durchmesser, aufgesteckt werden, wobei der freie Innendurchmesser der Abschnitte 3 und 4 vorzugsweise etwas kleiner ist als der Außendurchmesser des Rohrstutzens, auf welchen dieser Anschlussabschnitt aufgesteckt wird, damit er unter leichter elastischer Spannung dicht an der Außenseite des Rohrstutzens anliegt. Darüber kann auch noch ein Spannring oder Spannband angeordnet werden, der den Ballon sicher an dem Rohrstutzen festhält, wobei der Flanschabschnitt 4 als Sicherung dient.

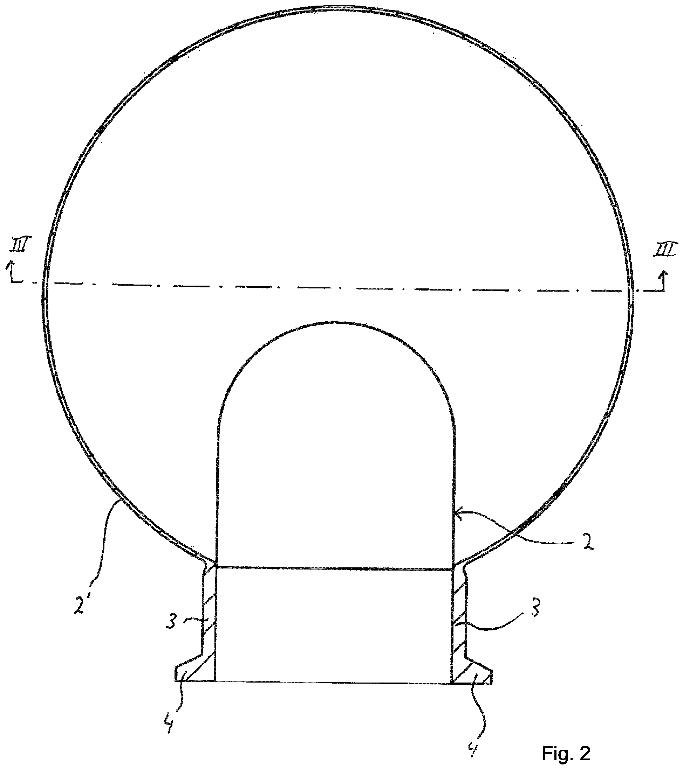

Figur 2 zeigt den Dehnungsabschnitt 2 in einem gedehnten Zustand, wobei der Dehnungsabschnitt im gedehnten Zustand mit 2' bezeichnet ist. Gleichzeitig ist auch die Kontur des ungedehnten Abschnittes 2 eingezeichnet. Die Größenverhältnisse zwischen dem Dehnungsabschnitt 2' im gedehnten und im drucklosen Zustand 2 gibt in etwa realistisch das Größenverhältnis in einem typischen Regelbereich wieder, in welchem der Druck im Inneren des Ausgleichsballons 2 unabhängig vom Volumen im Wesentlichen konstant bleibt. Mit anderen Worten, eine sehr geringfügige Erhöhung des Drucks führt sofort zu einer entsprechenden Dehnung des Abschnittes 2', die den Druck wieder auf den Regelwert absenkt. Umgekehrt führt eine geringfügige Drucksenkung bereits zu einer Reduzierung des Volumens des Abschnittes 2', sodass auch dann wieder der Regeldruck erreicht wird.

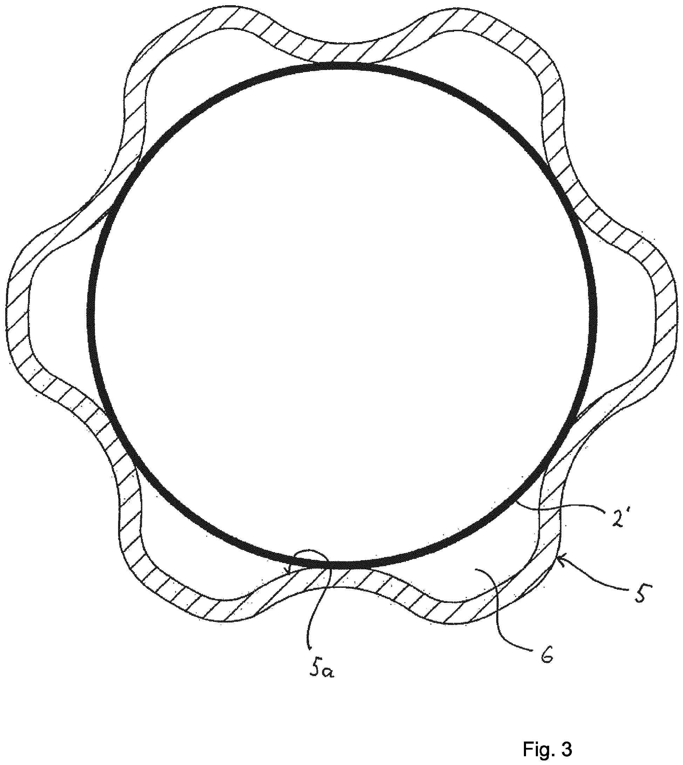

Figur 3 zeigt eine besondere Ausführungsform der vorliegenden Erfindung mit einer Schutzhülle 5, die aus einem relativ festen und im Vergleich zu dem Druckausgleichsballon 1 praktisch nicht dehnbaren Material besteht, welches vorzugsweise durchsichtig ist, sodass man den konkreten Zustand des Druckausgleichsballons 1 durch die Schutzhülle 5 hindurch sehen kann.

Im Allgemeinen könnte die Schutzhülle 5 kugelförmig sein mit einem Öffnungsabschnitt, welcher dem Anschlussabschnitt 3, 4 des Druckausgleichsballons 1 entspricht, sodass sich der Dehnungsabschnitt 2' bei Erreichen eines Radius, welcher dem Innenradius der Schutzhülle entspricht, vollflächig an die Innenwand der Schutzhülle anlegen würde, sodass im Falle einer weiteren Gaszufuhr in das Innere des Ballons der Druck sofort und im Wesentlichen proportional zur Zunahme der Gasmenge ansteigen würde.

Die Schutzhülle 5 gemäß Figur 3 weicht jedoch von einer Kugelform dadurch ab, dass sie von dem unteren Öffnungsbereich ausgehende, streifenförmige Ausbuchtungen 6 aufweist, wie man im Schnittbild der Figur 3 sehr deutlich erkennen kann, wohingegen ein Schnitt senkrecht zur Ebene der Figur 3 noch im Wesentlichen eine Kugelform bzw. von der Innenseite her rein konkave Form der Schutzhülle 5 wiedergeben würde. Diese Ausgestaltung führt dazu, dass der Dehnungsabschnitt 2' nach Erreichen eines Radius, welcher dem Radius der konvexen Abschnitte der Schutzhülle 5 entspricht, sich an die Innenwand 5a dieser konvexen Abschnitte anlegt, die in diesem Bereich eine weitere Dehnung des Abschnittes 2' nicht mehr zulassen. Bei weiterer Gaszufuhr bzw. Erhöhung des Druckes muss sich der Dehnungsabschnitt daher in die Ausbuchtungen 6 hinein ausdehnen. Diese Einschränkung der Dehnung des Abschnittes 2' entlang der Wände 5a der konvexen Bereiche der Schutzhülle führt zu einer veränderten Dehnungs-/Druckcharakteristik des Druckausgleichsballons 1, sodass, nachdem der Ballon ein Volumen erreicht hat, bei welchem der Dehnungsabschnitt an den Wandabschnitten 5a anliegt, der Druck bei weiterer Gaszufuhr nicht mehr konstant bleibt, sondern leicht linear ansteigt. Auf diese Weise ist es möglich, in relativ kontrollierter Weise den Druck von dem normalen Regeldruck ausgehend, allmählich und kontinuierlich auf einen Druck zu erhöhen, wie er nach dem Ausfüllen der Ausbuchtungen 6 durch die sich darin ausdehnenden Teile des Dehnungsabschnittes 2' ausgefüllt sind. Dies ermöglicht eine deutlich erleichterte Einstellung eines bestimmten Druckwertes zwischen dem Regeldruck und dem erwähnten Enddruck, der beispielsweise in etwa das Doppelte des Regeldruckes betragen kann. Wenn also der Regeldruck im Bereich von 20 bis 25 mbar liegt, könnte der Enddruck zwischen 40 und 50 mbar liegen, wobei sich dieser Enddruck durch entsprechende Formgebung der Ausbuchtungen 6 und der dazwischen liegenden konvexen Abschnitte in größeren Bereichen variieren lässt.

Ein entsprechend höherer Druck des Coughs einer Beatmungskanüle ist beispielsweise erforderlich, um Blutungen zu stillen oder wenn bestimmte Behandlungen oder technische Maßnahmen am Patienten oder einer Beatmungskanüle zu treffen sind, die unvermeidlich mit stärkeren Bewegungen der Beatmungskanüle verbunden sind, wobei aber die Dichtigkeit des Coughs in der Trachea nicht beeinträchtigt werden soll.

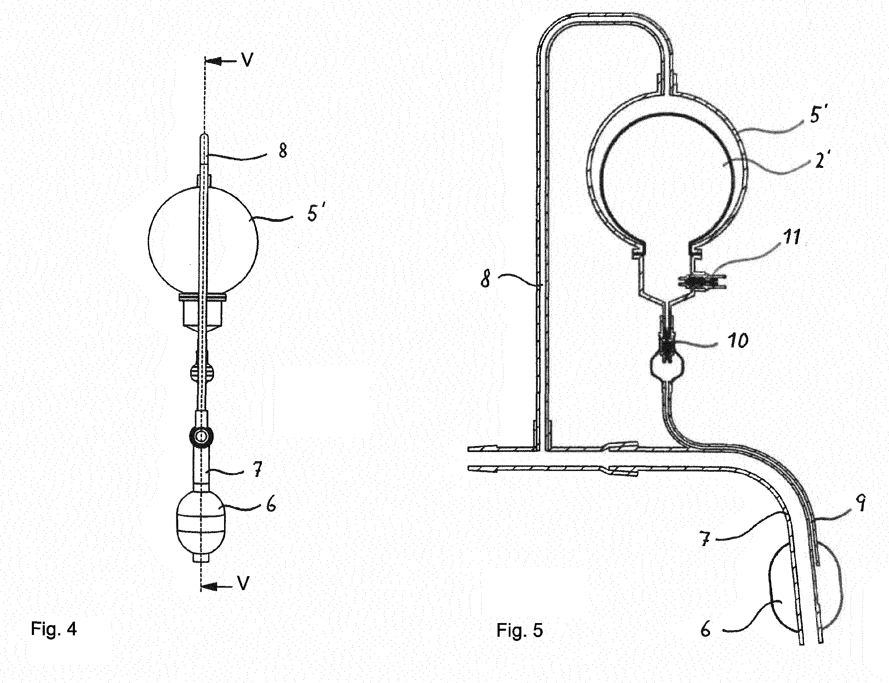

Figur 4 zeigt schematisch ein komplettes Beatmungssystem bestehend aus einer Beatmungskanüle 7 mit einem Cuff 6 und einem in einer kugelförmigen Schutzhülle 5' aufgenommenen Druckausgleichsballon. Die Details dieser Ausführungsform sind noch besser in der Schnittansicht gemäß Figur 5 zu erkennen. Figur 5 zeigt schematisch die Beatmungskanüle 7, den die Beatmungskanüle 7 umgebenden Cuff 6, der der Abdichtung des Zwischenraums zwischen der Wand der Trachea und der Außenseite der Kanüle 7 dient, mit einem Füllschlauch 9, der das Innere des Cuffs 6 mit einem Druckausgleichsballon 2' verbindet, wobei in diesem Fall zusätzlich noch ein Dämpfungsventil 10 in dem Füllschlauch 9 bzw. dessen Verbindung zu dem Druckausgleichsballon 2' vorgesehen ist, das dazu dient, den etwaigen Luft- bzw. Gasstrom aus dem Cuff 6 in den Ausgleichsballon 2' zu dämpfen. Bei der Beatmung und auch bei der Spontanatmung variiert der Druck in der Lunge und der Trachea des Patienten, wodurch wiederum von außen Druck auf den Cuff 6 ausgeübt wird, der dadurch Luft über den Füllschlauch 9 in den Ausgleichsballon 2' drückt. Dabei besteht die Gefahr, dass der Cuff 6 nicht mehr dicht an der Wand der Trachea anliegt.

Stattdessen sollte die Luft bzw. das Füllgas in dem Cuff 6 etwas länger verbleiben, wobei auch eine kurzzeitige Druckerhöhung in Kauf genommen wird, damit während einer Atemdruckspitze die Dichtigkeit der Anlage des Cuffs 6 an der Wand der Trachea gewährleistet bleibt. Das Dämpfungsventil 10 sorgt dafür, dass der Druck in dem Cuff 6 zumindest kurzzeitig, d.h. während entsprechender Atemdruckspitzen, höher sein kann als der Druck im Ausgleichsballon. In umgekehrter Richtung ist das Dämpfungsventil 10 jedoch durchlässig, sodass ein Nachlassen des Drucks im Cuff 6 unmittelbar ausgeglichen wird. Das Ventil 11 dient zur Befüllung des Cuffs und des Ausgleichsballons mit einer vorgegebenen Menge an Füllgas (zum Beispiel Luft oder Stickstoff).

Eine Alternative oder Ergänzung zu dem Dämpfungsventil 10 liegt bei dem erfindungsgemäßen Beatmungssystem in einem Verbindungsschlauch 8 zwischen der Beatmungskanüle 7 und der Schutzhülle 5', die bei dieser Ausführungsform auch kugelförmig ist bzw. sein kann.

Atemdruckspitzen, wie sie sowohl bei der künstlichen Beatmung als auch bei der Spontanatmung auftreten und die von außen auf den Cuff 6 wirken, liegen primär im Inneren der Beatmungskanüle 7 vor, sodass die entsprechenden Druckspitzen auch über den Verbindungsschlauch 8 in das Innere der Schutzhülle 5' übertragen werden und damit auch von außen auf den Ausgleichsballon 2' wirken. Durch die gleichzeitige Druckbeaufschlagung sowohl des Ausgleichsballons 2' als auch des Cuffs 6 von außen bleibt das System insgesamt besser im Gleichgewicht.

Allerdings bedeutet dies, dass der Innendruck in dem Cuff zusätzlich um den vollen Druck einer Atemluftspitze ansteigt. Falls also der Atemdruck 20 mbar beträgt und auch der Cuffdruck 20 mbar und der Atemdruck über den Verbindungsschlauch 8 in die Schutzhülle 5' übertragen wird, steigt der Druck im Druckausgleichsballon 2' ebenso wie in dem Cuff 6 um 20 mbar an, sodass der Cuffdruck insgesamt bereits 40 mbar beträgt, was auf Dauer unerwünscht sein könnte.

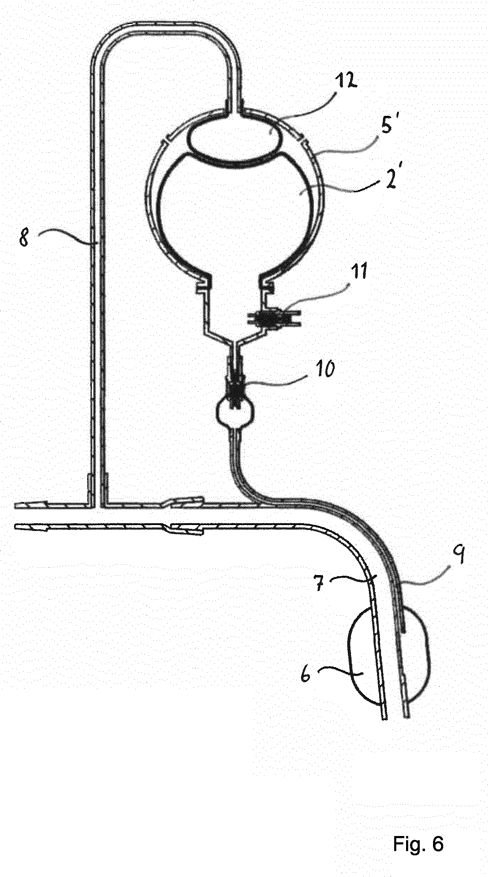

Um diesen Effekt etwas zu dämpfen, sieht die in Figur 6 dargestellte Ausführungsform noch eine Abwandlung dahingehend vor, dass nicht das gesamte Volumen der Schutzhülle 5' mit dem Atemdruck aus der Kanüle 7 beaufschlagt wird, sondern nur ein weiterer Übertragungsballon 12, der nur mit einem Teil der Oberfläche des Druckausgleichsballons 2' in Kontakt tritt, sodass der Druck im Inneren des Ausgleichsballons 2' nicht um den vollen Wert des Atemdrucks ansteigt.

Für Zwecke der ursprünglichen Offenbarung wird darauf hingewiesen, dass sämtliche Merkmale, wie sie sich aus der vorliegenden Beschreibung, den Zeichnungen und den abhängigen Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen und die Betonung der Unabhängigkeit der einzelnen Merkmale voneinander wird hier nur der Kürze und der Lesbarkeit der Beschreibung wegen verzichtet.

## Patentansprüche

1. Druckausgleichsvorrichtung für die Verwendung an Beatmungskanülen mit einem Druckausgleichsballon (1), dessen Volumen mit einem weiteren Volumen in Verbindung steht, dessen Druck auch bei einer aufgezwungenen Volumenänderung einen möglichst konstanten Wert behalten soll, und einer Schutzhülle (5) wobei der den Druck im Allgemeinen konstant haltende Ausgleichsballon in der Schutzhülle (5) aufgenommen ist, die abweichend von einer gleichmäßig konkaven, der äußeren Ballonkontur angepassten Form radiale Ausbuchtungen (6) aufweist, die so gestaltet sind, dass der Ausgleichsballon sich zunächst an der Kontur des Ballons angepasste Wandbereiche der Schutzhülle (5) anlegt und anschließend bei Druckerhöhung nur in die Ausbuchtungen (6) hinein ausdehnt, wodurch der Druck in dem Ausgleichsballon (1) mit der Volumenzunahme in die Ausbuchtungen hinein allmählich ansteigt, **dadurch gekennzeichnet, dass** die Schutzhülle (5) eine kugelförmige Grundform mit streifenförmigen Ausbuchtungen (6) aufweist.

2. Druckausgleichsvorrichtung nach Anspruch 1, **dadurch gekennzeichnet, dass** die das Volumen der Ausbuchtungen (6) der Schutzhülle (5) mindestens 5 % des Volumens der Schutzhülle (5) ohne die Ausbuchtungen (6) beträgt.

3. Druckausgleichsvorrichtung nach einem der vorstehenden Ansprüche, **dadurch gekennzeichnet, dass** der Druckausgleichsballon (1) aus einem styrolbasierten TPE, wie z. B. SBS (Styrol-Butadien-Styrol), SIS (Styrol-Isopren-Styrol), SEPS (Styrol-Ethylen-Propylen-Styrol) und insbesondere aus SEBS (Styrol-Ethylen-Butylen-Styrol) besteht.

4. Druckausgleichsvorrichtung nach einem der vorstehenden Ansprüche, **dadurch gekennzeichnet, dass** der Druckausgleichsballon (1) durch Spritzgießen hergestellt ist.

5. Druckausgleichsvorrichtung nach einem der vorstehenden Ansprüche, **dadurch gekennzeichnet, dass** die Wandstärke des drucklosen Ballons vordem erstmaligen Überdehnen zwischen 0,4 und 1,5 mm liegt.

6. Druckausgleichsvorrichtung nach einem der vorstehenden Ansprüche, **dadurch gekennzeichnet, dass** der Ballon (1) einen eine Ballonöffnung umgebenden verstärkten Öffnungsring aufweist, dessen Wandstärke mindestens 50% größer ist als die Wandstärke des Ballons (1) im Übrigen.

7. Druckausgleichsvorrichtung nach einem der vorstehenden Ansprüche, **dadurch gekennzeichnet, dass** die Maße des Öffnungsrings an einen Standardanschluss mit einem Durchmesser zwischen 12 und 25 mm angepasst sind.

8. Druckausgleichsvorrichtung nach einem der vorstehenden Ansprüche, **dadurch gekennzeichnet, dass** das Regelvolumen den Bereich von 10 bis 100 cm³ umfasst.

9. Druckausgleichsvorrichtung nach einem der vorstehenden Ansprüche, **dadurch gekennzeichnet, dass** das Regelvolumen den Bereich von 20 bis 80 cm³ umfasst.

10. Druckausgleichsvorrichtung nach einem der vorstehenden Ansprüche, **dadurch gekennzeichnet, dass** der Ballon (1) vor dem ersten Gebrauch einer Überdehnung auf ein Volumen ausgesetzt wurde, das mindestens das Vierfache des maximalen Regelvolumens beträgt.

11. Druckausgleichsvorrichtung nach einem der vorstehenden Ansprüche, **dadurch gekennzeichnet, dass** der Ballon auf einen Regelwert (Solldruckbereich) zwischen 20 und 30 mbar eingestellt ist.

## Claims

1. A pressure equalisation device for use on respiration cannulas comprising a pressure equalisation balloon (1) whose volume is connected to a further volume, the pressure of which is to maintain a value which is as constant as possible even in the event of a forced change in volume, and a protective case (5), wherein the equalisation balloon which keeps the pressure generally constant is accommodated in the protective case (5) which has radial outward bulges (6) deviating from a uniformly concave shape matched to the outside contour of the balloon, which bulges of such a configuration that the equalisation balloon firstly bears against wall regions of the protective case (5), that are matched to the contour of the balloon, and then upon an increase in pressure expands only into the outward bulges (6), whereby the pressure in the equalisation balloon (1) gradually increases with the increase in volume into the outward bulges, **characterised in that** the protective case (5) is of a spherical basic shape with strip-shaped outward bulges (6).

2. A pressure equalisation device according to claim 1 **characterised in that** the volume of the outward bulges (6) of the protective case (5) is at least 5% of the volume of the protective case (5) without the outward bulges (6).

3. A pressure equalisation device according to one of the preceding claims **characterised in that** the pressure equalisation balloon (1) comprises a styrene-based TPE like for example SBS (styrene-butadiene-styrene), SIS (styrene-isoprene-styrene), SEPS (styrene-ethylene-propylene-styrene) and in particular SEBS (styrene-ethylene-butylene-styrene).

4. A pressure equalisation device according to one of the preceding claims **characterised in that** the pressure equalisation balloon (1) is produced by injection moulding.

5. A pressure equalisation device according to one of the preceding claims **characterised in that** the wall thickness of the pressure-less balloon prior to the first-time overstretching is between 0.4 and 1.5 mm.

6. A pressure equalisation device according to one of the preceding claims **characterised in that** the balloon (1) has a reinforced opening ring which extends around a balloon opening and whose wall thickness is at least 50% greater than the wall thickness of the balloon (1) elsewhere.

7. A pressure equalisation device according to one of the preceding claims **characterised in that** the dimensions of the opening ring are matched to a standard connection of a diameter of between 12 and 25 mm.

8. A pressure equalisation device according to one of the preceding claims **characterised in that** the regulating volume includes the range of between 10 and 100 cm³.

9. A pressure equalisation device according to one of the preceding claims **characterised in that** the regulating volume includes the range of of 20 to 80 cm³.

10. A pressure equalisation device according to one of the preceding claims **characterised in that** the balloon (1) prior to the first use was subjected to an overstretching to a volume which is at least four times the maximum regulating volume.

11. A pressure equalisation device according to one of the preceding claims **characterised in that** the balloon is set to a regulating value (target pressure range) of between 20 and 30 mbars.

## Revendications

1. Dispositif de compensation de pression destiné à être utilisé au niveau de canules de ventilation, comportant un ballon de compensation de pression (1) dont le volume est lié à un autre volume dont la pression doit conserver une valeur la plus constante possible même lors d'une modification de volume imposée, et comportant une enveloppe de protection (5), dans lequel le ballon de compensation maintenant la pression généralement constante est logé dans l'enveloppe de protection (5) qui, en s'écartant d'une forme uniformément concave adaptée au contour extérieur du ballon, présente des renflements radiaux (6) conçus de façon telle que le ballon de compensation épouse initialement des régions de paroi de l'enveloppe de protection (5) adaptées au contour du ballon et s'étend ensuite, lorsque la pression augmente, uniquement au sein des renflements (6), ce qui fait que la pression au sein du ballon de compensation (1) augmente progressivement avec l'augmentation de volume au sein des renflements, **caractérisé en ce que** l'enveloppe de protection (5) présente une forme de base sphérique avec des renflements (6) en forme de bande.

2. Dispositif de compensation de pression selon la revendication 1, **caractérisé en ce que** le volume des renflements (6) de l'enveloppe de protection (5) correspond à au moins 5 % du volume de l'enveloppe de protection (5) sans les renflements (6).

3. Dispositif de compensation de pression selon l'une des revendications précédentes, **caractérisé en ce que** le ballon de compensation de pression (1) est constitué de TPE à base de styrène, par exemple de SBS (styrène-butadiène-styrène), de SIS (styrène-isoprène-styrène), de SEPS (styrène-éthylène-propylène-styrène) et en particulier de SEBS (styrène-éthylène-butylène-styrène).

4. Dispositif de compensation de pression selon l'une des revendications précédentes, **caractérisé en ce que** le ballon de compensation de pression (1) est réalisé par moulage par injection.

5. Dispositif de compensation de pression selon l'une des revendications précédentes, **caractérisé en ce que** l'épaisseur de paroi du ballon non pressurisé avant la première distension est comprise entre 0,4 et 1,5 mm.

6. Dispositif de compensation de pression selon l'une des revendications précédentes, **caractérisé en ce que** le ballon (1) présente une bague d'ouverture renforcée entourant une ouverture de ballon, dont l'épaisseur de paroi est d'au moins 50 % supérieure à l'épaisseur de paroi du reste du ballon (1).

7. Dispositif de compensation de pression selon l'une des revendications précédentes, **caractérisé en ce que** les cotes de la bague d'ouverture sont adaptées à un raccord standard ayant un diamètre compris entre 12 et 25 mm.

8. Dispositif de compensation de pression selon l'une des revendications précédentes, **caractérisé en ce que** le volume de régulation comprend la plage comprise entre 10 et 100 cm³.

9. Dispositif de compensation de pression selon l'une des revendications précédentes, **caractérisé en ce que** le volume de régulation comprend la plage comprise entre 20 et 80 cm³.

10. Dispositif de compensation de pression selon l'une des revendications précédentes, **caractérisé en ce que** le ballon (1) a été soumis avant la première utilisation à une distension jusqu'à un volume correspondant à au moins quatre fois le volume de régulation maximal.

11. Dispositif de compensation de pression selon l'une des revendications précédentes, **caractérisé en ce que** le ballon est réglé sur une valeur de régulation (plage de pression théorique) comprise entre 20 et 30 mbar.
